(19) Europäisches Patentamt
European Patent Office
Office européen des brevets

(11) Veröffentlichungsnummer : **0 014 454**
**B1**

(12) **EUROPÄISCHE PATENTSCHRIFT**

(45) Veröffentlichungstag der Patentschrift : **22.09.82**

(21) Anmeldenummer : **80100547.1**

(22) Anmeldetag : **04.02.80**

(51) Int. Cl.³ : **C 07 D223/16**, C 07 C 97/10, C 07 C 49/835, C 07 D209/48, C 07 C143/68// C07C49/784

# (54) Verfahren und Zwischenprodukte zur Herstellung von 2-Benzazepin-Derivaten.

(30) Priorität : **07.02.79 US 10118**
**22.01.80 CH 511/80**

(43) Veröffentlichungstag der Anmeldung : **20.08.80 (Patentblatt 80/17)**

(45) Bekanntmachung des Hinweises auf die Patenterteilung : **22.09.82 Patentblatt 82/38**

(84) Benannte Vertragsstaaten : **AT BE CH DE FR GB IT LU NL SE**

(56) Entgegenhaltungen :
**GB A 1 045 244**
**NL A 7 506 570**
**US A 3 420 817**
**US A 3 939 271**

(73) Patentinhaber : **F. HOFFMANN-LA ROCHE & CO. Aktiengesellschaft**
**CH-4002 Basel (CH)**

(72) Erfinder : **Trybulski, Eugene J.**
**345 Claremont Avenue**
**Montclair, NJ (US)**

(74) Vertreter : **Lederer, Franz, Dr. et al**
**Patentanwälte Dr. Franz Lederer Reiner F. Meyer**
**Lucile-Grahn-Strasse 22**
**D-8000 München 80 (DE)**

**EP 0 014 454 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 18, rue St-Denis, 75001 Paris, France

# Verfahren und Zwischenprodukte zur Herstellung von 2-Benzazepin-Derivaten

Die vorliegende Erfindung bezieht sich auf die Herstellung von Verbindungen der allgemeinen Formel

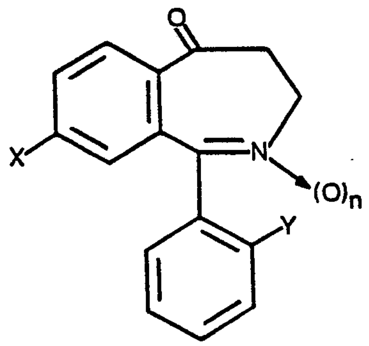

(I)

worin X und Y je Wasserstoff, Halogen mit einer Atomnummer von höchstens 35 oder Trifluormethyl und n 0 oder 1 bedeuten.

Diese Verbindungen sind wertvoll als Zwischenprodukte bei der Herstellung von pharmakologisch aktiven 2-Benzazepinen.

Im speziellen betrifft die vorliegende Erfindung ein neues Verfahren zur Herstellung der Verbindungen der obigen Formel I und neue Zwischenprodukte für deren Herstellung.

Gemäss dem Verfahrensaspekt der vorliegenden Erfindung können die Verbindungen der obigen Formel I dadurch hergestellt werden, dass man eine Verbindung der allgemeinen Formel

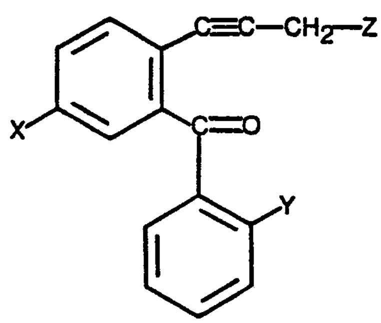

(II)

worin X und Y obige Bedeutung besitzen und Z eine Hydroxyamino-, eine Amino- oder eine geschützte Aminogruppe bedeutet, unter sauren Bedingungen mit einem Quecksilber-(II)-Salz oder in Gegenwart von Wasser mit einer starken Säure behandelt, falls Z eine geschützte Aminogruppe ist, die Schutzgruppe abspaltet und, falls eine Verbindung der Formel I, worin n 0 ist, erhalten wird, diese Verbindung erwünschtenfalls zu einer Verbindung der Formel I, worin n 1 ist, oxidiert.

In ihrem Stoffaspekt betrifft die vorliegende Erfindung Verbindungen der obigen allgemeinen Formel II.

Die Verbindungen der Formel II sind nützlich als Zwischenprodukte bei der Herstellung von pharmakologisch aktiven 2-Benzazepinen.

In der vorliegenden Beschreibung bezeichnen die Ausdrücke « Halogen » oder « Halo » die drei Formen Chlor, Brom oder Fluor. Der Ausdruck « geschützte Aminogruppe » umfasst acylierte Aminogruppen, wie Phthalimido, Carbobenzoxyamino, tert. Butyloxycarbonylamino, Formylamino, Acetylamino und Trifluoracetylamino.

Der Ausdruck « Abgangsgruppe » umfasst Alkyl- und Arylsulfonyloxygruppen (wie Methansulfonyloxy und Toluolsulfonyloxy), Brom und Chlor.

Durch die nachfolgenden Reaktionsschemata, worin X und Y obige Bedeutung haben, wird die vorliegende Erfindung näher illustriert :

Schema 1

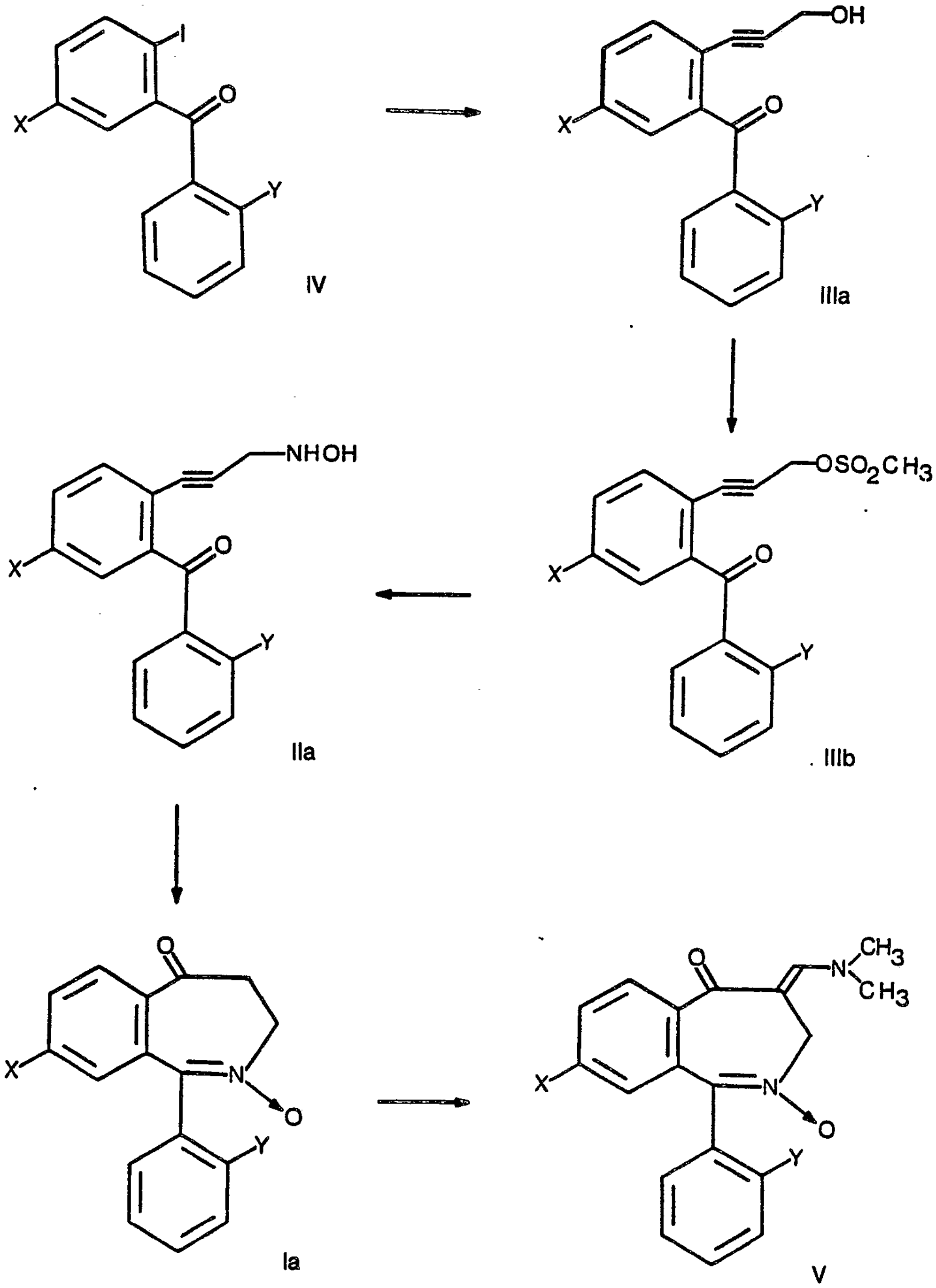

IV, IIIa, IIIb, IIa, Ia, V

Schema 2

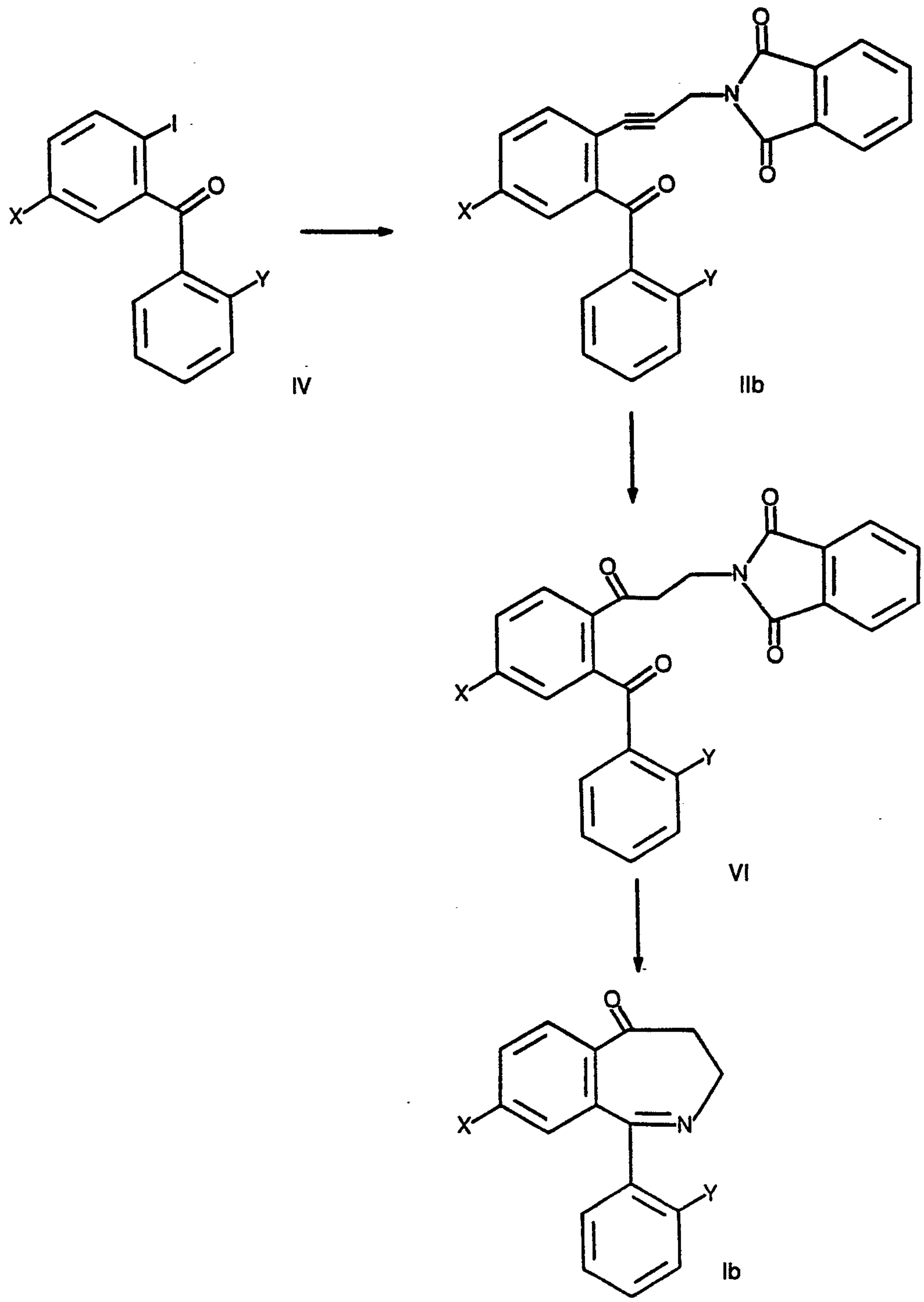

IV

IIb

VI

Ib

Schema 3

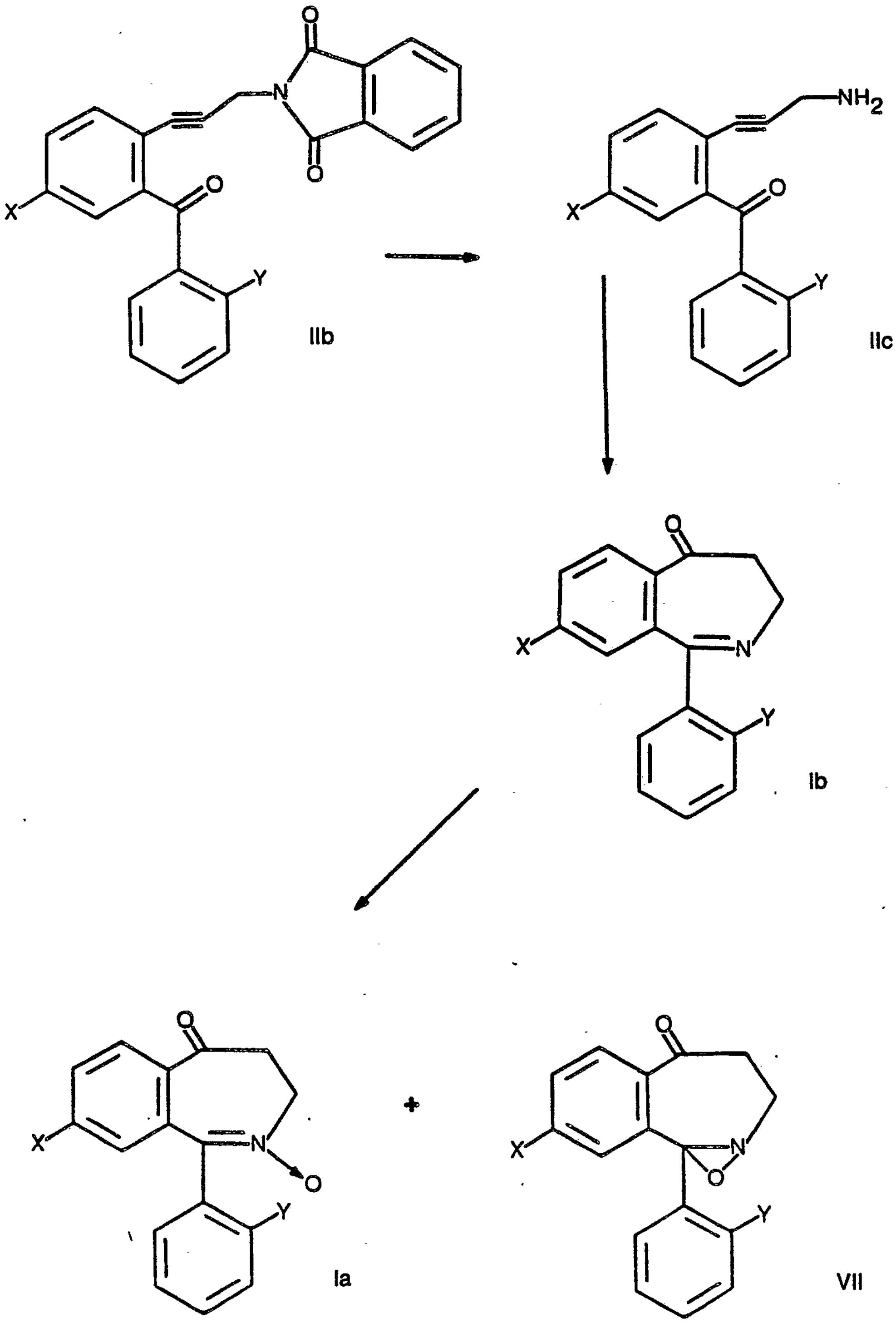

IIb

IIc

Ib

Ia

+

VII

.Schema 1

IV ⟶ IIIa

Die Verbindung der Formel IIIa kann erhalten werden, indem man eine Verbindung der Formel IV in Gegenwart eines Palladium-(II)-Salzes (wie Palladiumchlorid oder Palladiumacetat), eines Organophosphins (z. B. Triphenylphosphin), Kupfer-(I)-Jodid und eines tertiären oder vorzugsweise sekundären Amins (wie Diäthylamin oder Diisopropylamin) mit Propargylalkohol zur Reaktion bringt. Als Lösungsmittel für diese Reaktion kann das Amin selbst (z. B. Diäthylamin), ein halogenierter Kohlenwasserstoff (z. B. Methylenchlorid), Dimethylformamid oder ein ätherisches Lösungsmittel dienen. Die Reaktionstemperatur kann in einem Bereich von 0 °C bis 70 °C liegen, wobei Raumtemperatur bevorzugt ist. Die Anwesenheit von Kupfer-(I)-Jodid ist zwingend, wenn die Reaktion bei Raumtemperatur oder unterhalb davon durchgeführt wird, wogegen dies nicht der Fall ist, wenn die Reaktion unter Erhitzen durchgeführt wird. Die Anwesenheit des Organophosphins ist nicht absolut notwendig, jedoch höchst vorteilhaft. Anstelle von Palladium-(II)-Salz plus Organophosphin kann auch ein geeigneter Komplex verwendet werden, wie z. B. Dichlor-bis-(triphenylphosphin)-palladium-(II).

Die Ausgangsprodukte der Formel IV können dadurch hergestellt werden, dass man ein entsprechendes bekanntes Aminobenzophenon mittels Natriumnitrit in Schwefelsäure diazotiert und die Salze durch Ausfällung der entsprechenden Tetrafluoroborate isoliert, worauf man diese Tetrafluoroborate in Wasser suspendiert und mit wässrigem Kaliumjodid behandelt, um so das Jodbenzophenon zu erhalten. Diese Reaktionen werden unter Verwendung an sich bekannter Methoden durchgeführt.

IIIa ⟶ IIIb

Die Verbindung der Formel IIIa kann in Gegenwart eines tertiären Amins, wie Triäthylamin, und in Gegenwart eines organischen Lösungsmittels, wie halogenierte Kohlenwasserstoffe (z. B. Methylenchlorid), Toluol oder Diäthyläther mit Methansulfonylchlorid umgesetzt werden. Die Reaktion kann zwischen − 78 °C und Raumtemperatur durchgeführt werden, wobei Temperaturen um 0 °C bevorzugt sind. Verbindungen entsprechend denjenigen der Formel IIIb, welche jedoch eine von Mesyloxy verschiedene geeignete Abgangsgruppe enthalten, können ebenfalls ausgehend von Verbindungen der Formel IIIa nach an sich bekannten Methoden hergestellt werden, beispielsweise mittels Toluolsulfonylchlorid, Thionylchlorid und Phosphortribromid.

IIIb ⟶ IIa ⟶ Ia

Die Verbindung der Formel IIIb oder ein Analogon davon, welches eine von Mesyloxy verschiedene geeignete Abgangsgruppe enthält, wird mit Hydroxylamin zur Reaktion gebracht. Dies erfolgt zweckmässigerweise in einem Alkohol ($C_1$ bis $C_4$) als Lösungsmittel und bei einer Reaktionstemperatur von 0 °C bis Raumtemperatur, wobei etwa Raumtemperatur bevorzugt ist. Das isolierte Zwischenprodukt IIa kann dann erfindungsgemäss in Gegenwart einer Säure, beispielsweise einer Carbonsäure oder einer verdünnten oder konzentrierten Mineralsäure, und zweckmässigerweise in Gegenwart eines inerten organischen Lösungsmittels mit einem Quecksilber-(II)-Salz zur Reaktion gebracht werden, beispielsweise mit dem Sulfat, Chlorid, Acetat, Trifluoracetat oder einem Sulfonat (einschliesslich Salze mit Sulfonsäuregruppen enthaltenden Ionenaustauschern). So kann die Verbindung der Formel IIa in einem inerten Lösungsmittel, z. B. einem halogenierten Kohlenwasserstoff, wie Methylenchlorid, mit einer Mischung von Quecksilber-(II)-Sulfat und einer Carbonsäure ($C_1$ bis $C_5$), wie Ameinsensäure oder Essigsäure, zur Reaktion gebracht werden. Die Reaktionstemperatur liegt zwischen − 10 °C und Raumtemperatur, vorzugsweise zwischen 0 °C und 5 °C. Andererseits kann die Verbindung der Formel IIa erfindungsgemäss auch in Gegenwart von Wasser mit einer starken Säure behandelt werden, beispielsweise mit konzentrierter Schwefelsäure bei − 10 °C bis Raumtemperatur, vorzugsweise bei 0 °C.

Die Hydrataticn von Alkynverbindungen mittels Quecksilber-(II)-Salzen oder starker Säure ist an sich bekannt, vgl. GB-A 1.045.244 und « Survey of Organic Syntheses » von Calvin A. Buehler und Donald E. Pearson, Seite 670.

Ia ⟶ V

Die Verbindung der Formel Ia kann dann in einem inerten Lösungsmittel, beispielsweise einem halogenierten Kohlenwasserstoff (wie Methylenchlorid), Dimethylformamid oder einem hochsiedenden Aether, mit Dimethylformamiddimethylacetal zur Reaktion gebracht werden. Die Reaktionstemperatur kann zwischen 0 °C und 100 °C liegen, wobei Raumtemperatur bevorzugt ist.

Schema 2

IV ⟶ IIb

Die Verbindung der Formel IV kann in Gegenwart von Palladiumchlorid, Kupfer-(I)-jodid, eines Organophosphins (z. B. Triphenylphosphin) und eines sekundären Amins, wie Diäthylamin oder Diisopropylamin, mit Propargylphthalimid umgesetzt werden. Die Lösungsmittel und Reaktionsbedingungen sind wie oben für Stufe IV → IIIa beschrieben. Verbindungen entsprechend denjenigen der Formel IIb, welche jedoch eine von Phthalimido verschiedene geeignete geschützte Aminogruppe enthalten, können in ähnlicher Weise ebenfalls ausgehend von Verbindungen der Formel IV hergestellt werden, beispielsweise mittels N-Carbobenzoxypropargylamin oder entsprechenden N-Formyl-, N-tert. Butoxycarbonyl-, N-Acetyl- oder N-Trifluoracetylverbindungen.

IIb ⟶ VI

Die Verbindung der Formel IIb oder ein Analogon davon, welches eine von Phthalimido verschiedene geeignete geschützte Aminogruppe enthält, kann dann erfindungsgemäss Reaktionsbedingungen unterworfen werden, wie sie oben für Stufe IIa → Ia beschrieben sind. So kann man sie in Gegenwart eines halogenierten Kohlenwasserstoffs, wie Methylenchlorid, oder eines inerten Aethers mit einer Mischung von Quecksilber-(II)-Sulfat und einer Carbonsäure ($C_1$ bis $C_5$), wie z. B. Ameisensäure oder Essigsäure, zur Reaktion bringen. Die Reaktionstemperatur kann zwischen 0 °C und Raumtemperatur liegen, wobei 0 °C bevorzugt ist. Andererseits kann die Verbindung der Formel IIb oder ein Analogon davon, welches eine von Phthalimido verschiedene geeignete geschützte Aminogruppe enthält, bei −10 °C bis Raumtemperatur, vorzugsweise bei 0 °C, mit konzentrierter Schwefelsäure behandelt werden.

VI ⟶ Ib

Um eine Verbindung der Formel Ib zu erhalten, entfernt man erfindungsgemäss die Schutzgruppe von einer Verbindung der Formel VI oder von einem Analogon davon, welches eine von Phthalimido verschiedene geeignete geschützte Aminogruppe enthält.

Eine Verbindung der Formel VI wird zweckmässigerweise in einem Alkohol ($C_1$ bis $C_4$) als Lösungsmittel mit einem primären Alkylamin, wie Methylamin oder Aethylamin, zur Reaktion gebracht. Die Reaktionstemperatur kann zwischen 0 °C und Raumtemperatur liegen, wobei Raumtemperatur bevorzugt ist.

Eine andere Methode zur Herstellung der Verbindung der Formel Ib besteht in der Reaktion der Verdinbung der Formel VI mit Hydrazin in einem inerten Lösungsmittel, wie Aethanol, Mischungen von Aethanol und Chloroform, Tetrahydrofuran oder wässriges Aethanol. Die Reaktionstemperatur kann zwischen etwa Raumtemperatur und 100 °C variieren, wobei die Rückflusstemperatur des verwendeten Lösungsmittels bevorzugt ist. Das Produkt wird mit verdünnter Mineralsäure extrahiert und anschliessend durch Neutralisation isoliert.

Eine dritte Methode, welche zur Herstellung der Verbindung der Formel Ib verwendet werden kann, besteht in einer sauren oder alkalischen Hydrolyse der Verbindung der Formel VI. Für eine saure Hydrolyse kann man eine 30 % ige Lösung einer Mineralsäure, wie Chlorwasserstoffsäure, Bromwasserstoffsäure, Schwefelsäure oder Phosphorsäure verwenden, wobei die Reaktion bei oder etwa Rückflusstemperatur erfolgt. Für eine alkalische Hydrolyse verwendet man ein Alkalimetellhydroxyd, wie Kaliumhydroxyd oder Natriumhydroxyd. Inerte organische Lösungsmittel, wie diejenigen welche oben angeführt sind, können zur Solubilisierung der Ingredientien verwendet werden. Die Reaktion wird bei oder oberhalb der Rückflusstemperatur des verwendeten Lösungsmittels durchgeführt.

Methoden zur Abspaltung der Schutzgruppe von Verbindungen entsprechend denjenigen der Formel VI, welche jedoch eine von Phthalimido verschiedene geeignete geschützte Aminogruppe enthalten, sind jedem Fachmann leicht zugänglich.

Schema 3

IIb ⟶ IIc

Um eine Verbindung der Formel IIc zu erhalten, entfernt man die Schutzgruppe von einer Verbindung der Formel IIb oder von einem Analogon davon, welches eine von Phthalimido verschiedene geeignete geschützte Aminogruppe enthält, und zwar unter Reaktionsbedingungen, wie sie oben für Stuffe VI → Ib beschrieben sind. So kann eine Verbindung der Formel IIb in einem mit Wasser mischbaren Lösungsmittel, z. B. in Alkoholen ($C_1$ bis $C_4$), in Aethern oder in Dimethylformamid mit einem primären Alkylamin, wie Methylamin oder Aethylamin, zur Reaktion gebracht werden. Die Reaktionstemperatur kann zwischen 0 °C und 60 °C, vorzugsweise bei Raumtemperatur liegen.

IIc ⟶ Ib

Die Verbindung der Formel IIc kann dann erfindungsgemäss Reaktionsbedingungen unterworfen werden, wie sie oben für Stufe IIa → Ia beschrieben sind. So kann sie in Gegenwart eines halogenierten Kohlenwasserstoffs als Lösungsmittel mit einer Mischung von Quecksilber-(II)-Sulfat und einer Carbon-

säure ($C_1$ bis $C_5$) zur Reaktion gebracht werden oder kann bei Temperaturen zwischen − 10 °C und Raumtemperatur, vorzugsweise 0 °C, mit konzentrierter Schwefelsäure zur Reaktion gebracht werden.

Ib ⟶ Ia und VII

Die Verbindung der Formel Ib kann erpindungsgemäss in einem inerten organischen Lösungsmittel, beispielsweise einem halogenierten Kohlenwasserstoff, wie Methylenchlorid, oder einem Aether mit einer Persäure, wie Metachlorperbenzoesäure, behandelt werden. Die Reaktion kann bei 0 °C bis 40 °C durchgeführt werden, wobei Raumtemperatur bevorzugt ist. Das Produktegemisch kann anschliessend durch fraktionierte Kristallisation aufgetrennt werden ; die dünnschichtchromatographische Analyse zeigt die Gegenwart beider Produkte an.

Bezüglich des vorstehenden Schemas ist noch zu bemerken, dass die Ausdrücke « niederes Alkyl » oder « Alkyl » eine gerade oder verzweigte aliphatische Kohlenwasserstoffkette mit 1 bis 7, vorzugsweise 1 bis 4, Kohlenstoffatomen bezeichnen.

Die Verbindung der Formel Ib ist ein bekanntes Zwischenprodukt und ist nützlich in der Herstellung verschiedener Benzazepine, welche pharmakologisch verwertbar sind, nämlich als Anxiolytika, siehe beispielsweise U. S. Patentschriften Nos. 4.028.381, 4.022.800 und 4.022.801. Das vorliegende Verfahren zur Herstellung von Ib zeigt im Vergleich zum Stand der Technik insofern Vorteile, als es weniger Stufen umfasst, billiger ist und bessere Ausbeuten ergibt. In ähnlicher Weise wie die Verbindungen der Formel Ia können auch die Verbindungen der Formel Ib mit Dimethylformamid-dimethylacetal wie für Stufe Ia → V beschrieben, zur Reaktion gebracht werden.

Die Verbindung der Formel V, welche aus der Verbindung der Formel Ia hergestellt werden kann, ist ebenfalls ein nützliches Zwischenprodukt für die Herstellung von 2-Benzazepinen, beispielsweise von Pyrimido-2-benzazepinen der Formel

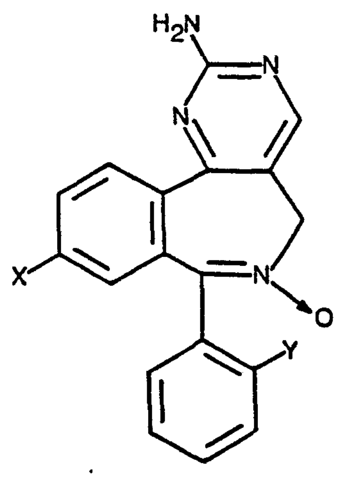

VIII

worin X und Y obige Bedeutung besitzen.

Die Verbindung der obigen Formel VIII kann dadurch hergestellt werden, dass man die Verbindung der Formel V mit einer Verbindung der Formel

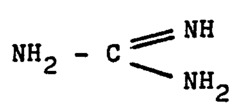

(IX)

zur Reaktion bringt. Hierfür kann irgendein inertes organisches Lösungsmittel verwendet werden, wie Methylenchlorid, Alkohole (wie Methanol), Aether (wie Dioxan, Tetrahydrofuran) oder Dimethylformamid ; die Reaktionstemperatur kann zwischen etwa Raumtemperatur und Rückflusstemperatur des verwendeten Lösungsmittels liegen, wobei etwa Raumtemperatur bevorzugt ist.

Die obige Reaktion von V mit IX zu VIII ist nicht Gegenstand der vorliegenden Erfindung, sondern wird zur Vervollständigung der Offenbarung bezüglich der Verwendbarkeit der Verbindungen der Formel Ia beschrieben. Die Pyrimido-2-benzazepine der Formel VIII entfalten anxiolytische und sedative Aktivitäten.

In den nachfolgenden Beispielen sind alle Temperaturen in Celsiusgraden angegeben. Beispiele 1 bis 18 illustrieren die Herstellung von Ausgangsprodukten, Beispiele 19 bis 26 das erfindungsgemässe Verfahren und Beispiele 29 bis 35 die Weiterverarbeitung von Endprodukten des erfindungsgemässen Verfahrens.

## Beispiel 1

Eine Mischung aus 76 g (1,1 Mol) Natriumnitrit und 450 ml Schwefelsäure wird auf dem Dampfbad auf

ca. 80° erwärmt bis vollständige Lösung eintritt. Nach Abkühlen auf 30° werden 232 g (1,0 Mol) 2-Amino-5-chlorbenzophenon unter Rühren portionenweise zugegeben, so dass die Temperatur zwischen 30 und 40° bleibt. Nach einer Stunde wird die Mischung langsam zu 3 Liter Eiswasser gegeben und über Hy-Flo filtriert. Das Filtrat wird unter Rühren langsam mit einer Lösung von 200 g (1,83 Mol) Natriumtetrafluoroborat in 800 ml Wasser versetzt. Der dabei entstehende Niederschlag wird abfiltriert und 2mal mit je 100 ml Wasser gewaschen.

Das feuchte 2-Benzoyl-4-chlorbenzoldiazoniumtetrafluoroborat wird in 3 Liter Wasser suspendiert und tropfenweise mit einer Lösung von 332 g (2 Mol) Kaliumjodid in 1 Liter Wasser versetzt. Die Mischung wird während 4 Stunden bei Raumtemperatur gerührt ; anschliessend filtriert man das Rohprodukt ab und gibt es zu 1 Liter siedendem Aether. Nach Filtrieren und Trocknen über Natriumsulfat wird die ätherische Lösung auf 500 ml eingeengt. Durch Zugeben von 100 ml Petroläther erhält man 5-Chlor-2-jod-benzophenon. Eine Probe des Materials wird aus Aether/Petroläther umkristallisiert und ergibt feine gelbe Prismen vom Smp. 80-82°.

## Beispiel 2

Die Herstellung von 5-Chlor-2'-fluor-2-jod-benzophenon erfolgt auf die gleiche Weise wie die Herstellung von 5-Chlor-2-jod-benzophenon und ergibt hellgelbe Prismen vom Smp. 78-81°.

## Beispiel 3

Methode A : Die Herstellung von 2',5-Dichlor-2-jod-benzophenon erfolgt auf die gleiche Weise wie die Herstellung von 5-Chlor-2-jod-benzophenon und ergibt hellgelbe Prismen vom Smp. 64-66°.

Methode B : Eine auf 0° gekühlte Lösung von 27 g (0,1 Mol) 2-Amino-2',5-dichlor-benzophenon in 50 ml Essigsäure und 30 ml Schwefelsäure wird unter Rühren tropfenweise mit 9,0 g (0,13 Mol) Natriumnitrit in 30 ml Wasser versetzt. Nach 1,5 Stunden tropft man eine Lösung von 33 g (0,2 Mol) Kaliumjodid in 40 ml Wasser langsam zu und rührt 2 Stunden bei 0°. Anschliessend verdünnt man mit Wasser und extrahiert mit Aether. Die ätherischen Auszüge werden mit 5 % iger Natriumthiosulfatlösung gewaschen, über Natriumsulfat getrocknet und im Vakuum eingedampft. Kristallisation des verbleibenden braunen Oels aus kaltem Aether ergibt 2',5-Dichlor-2-jod-benzophenon vom Smp. 63-65°, das in allen Belangen identisch mit authentischem Material ist.

## Beispiel 4

Die Herstellung von 2'-Chlor-2-jod-benzophenon erfolgt auf die gleiche Weise wie die Herstellung von 5-Chlor-2-jod-benzophenon und ergibt schwach gelbe Prismen vom Smp. 62-64°.

## Beispiel 5

Die Herstellung von 2-Jod-benzophenon erfolgt auf die gleiche Weise wie die Herstellung von 5-Chlor-2-jod-benzophenon und ergibt ein braunes Oel. Durch Reinigung einer Probe mittels Säulenchromatographie erhält man weisse Prismen vom Smp. 29-31°.

## Beispiel 6

Eine Mischung aus 0,71 g (4,0 mMol) Palladiumchlorid, 2,1 g (8,0 mMol) Triphenylphosphin, 0,80 g (4,2 mMol) Kupferjodid, 68,8 g (0,21 Mol) 5-Chlor-2-jodbenzophenon, 200 ml Diäthylamin und 400 ml Methylenchlorid wird bei Raumtemperatur unter Argon gerührt bis alles in Lösung ist. In einer Portion gibt man 40,0 g (0,22 Mol) N-Propargyl-phthalimid hinzu und rührt die entstehende Mischung während 20 Stunden. Nach Entfernen der flüchtigen Anteile im Vakuum, wird der Rückstand mit 200 ml Isopropanol verrieben. Durch Abfiltrieren des entstandenen Niederschlages erhält man rohes 1-[4-Chlor-2-benzoyl-phenyl]-3-phthalimidopropin vom Smp. 130-133°. Umkristallisation aus Aceton ergibt crèmefarbene Prismen vom Smp. 148-150°.

## Beispiel 7

Die Herstellung von 1-[4-Chlor-(2-fluorbenzoyl)-phenyl]-3-phthalimidopropin erfolgt auf die gleiche Weise wie die Herstellung von 1-[4-Chlor-2-benzoylphenyl]-3-phthalimidopropin und ergibt crèmefarbene Prismen vom Smp. 158-161°.

## Beispiel 8

Die Herstellung von 1-[4-Chlor-2-(2-chlorbenzoyl)-phenyl]-3-phthalimidopropin erfolgt auf die gleiche Weise wie die Herstellung von 1-[4-Chlor-2-benzoylphenyl]-3-phthalimidopropin und ergibt crèmefarbene Prismen vom Smp. 144-145°.

Beispiel 9

Die Herstellung von 1-[2-(2-Chlorbenzoyl) phenyl]-3-phthalimidopropin erfolgt auf die gleiche Weise wie die Herstellung von 1-[4-Chlor-2-benzoylphenyl]-3-phthalimidopropin und ergibt crèmefarbene Prismen vom Smp. 149-150°.

Beispiel 10

Die Herstellung von 1-[2-Benzoylphenyl]-3-phthalimidopropin erfolgt auf die gleiche Weise wie die Herstellung von 1-[4-Chlor-2-benzoylphenyl]-3-phthalimidopropin und ergibt crèmefarbene Prismen vom Smp. 164-165°.

Beispiel 11

Eine Mischung aus 0,37 g (0,5 mMol) Dichlor-bis-triphenylphosphin-Palladium II, 70 mg (0,35 mMol) Kupferjodid, 36,1 g (0,1 Mol) 5-Chlor-2'-fluor-2-jod-benzophenon, 12 ml (0,2 Mol) Propargylalkohol und 200 ml Diäthylamin wird bei Raumtemperatur während 4 Tagen gerührt. Nach Einengen der Mischung im Vakuum wird der Rückstand zwischen Aether und Wasser verteilt. Die ätherische Phase wird mit Wasser gewaschen, über Natriumsulfat getrocknet und im Vakuum eingedampft. Dabei erhält man 3-Hydroxy-1-[4-Chlor-2-(2-fluorbenzoyl) phenyl] propin als gelbbraunes Oel.

Beispiel 12

Zu einer auf 0° gekühlten Lösung von 28,9 g (0,1 Mol) 3-Hydroxy-1-[4-chlor-2-(2-fluorbenzoyl) phenyl] propin, 24,4 ml (0,175 Mol) Triäthylamin in 300 ml Methylenchlorid werden 13 ml (0,17 Mol) Methansulfonylchlorid tropfenweise zugegeben. Die Mischung wird nacheinander mit Eiswasser, kalter 1N Salzsäure und kalter gesättigter Natriumbicarbonat-Lösung gewaschen und über Natriumsulfat getrocknet. Durch Eindampfen der organischen Phase erhält man ein braunes Oel, welches aus Aether zu einem gelben Festkörper kristallisiert. Durch Umkristallisation aus Aether/Petroläther erhält man 3-Hydroxy-1-[4-chlor-2-(2-fluorbenzoyl)-phenyl] propin-methansulfonat als farblose Prismen vom Smp. 95-96°.

Biespiel 13

Methode A: Eine Mischung aus 72 g (0,18 Mol) 1-[4-Chlor-2-benzoylphenyl)-3-phthalimidopropin, 90 ml 40 % ige Methylamin-Lösung und 300 ml Aethanol wird bei Raumtemperatur während 90 Minuten gerührt. Nach Verdünnen der Mischung mit 300 ml Aether wird der Niederschlag abfiltriert. Das Filtrat wird mit weiteren 300 ml Aether verdünnt, mit Wasser gewaschen und über Natriumsulfat getrocknet. Eindampfen der ätherischen Lösunf im Vakuum ergibt ein braunes Oel, welches nach Zerreiben mit Aether einen gelben Festkörper ergibt. Durch Umkristallisation aus Aether erhält man 3-Amino-1-[4-chlor-2-benzoylphenyl]-propin als schwach gelbe Prismen vom Smp. 68-69°.

Methode B: Eine Mischung aus 4 g (10 mMol) 1-[4-Chlor-2-benzoylphenyl]-3-phthalimidopropin, 0,6 g (16 mMol) 85 %igem Hydrazinhydrat in 150 ml 95 %igem Aethanol wird während 5,5 Stunden zum Sieden erhitzt. Nach dem Abkühlen werden unlösliche Anteile durch Filtration entfernt. Das Filtrat wird mit Wasser verdünnt, mit Salzsäure angesäuert und mit Aether ausgeschüttelt. Die wässrige Phase wird mit verdünnter Natriumcarbonat-Lösung basisch gestellt und mit Methylenchlorid extrahiert. Die Methylenchlorid-Lösung wird über Natriumsulfat getrocknet und im Vakuum zur Trockne eingedampft. Durch Umkristallisation des Rückstandes aus Aether/Petroläther erhält man 3-Amino-1-[4-chlor-2-benzoylphenyl] propin als schwach gelben Festkörper vom Smp. 68-69°, welcher in allen Belangen identisch mit authentischem Material ist.

Durch Behandeln einer Lösung von 3-Amino-1-[4-Chlor-2-benzoylphenyl] propin in Methanol mit 6 %iger methanolischer Salzsäure und Ausfällen des Produktes mit Aether erhält man das entsprechende rohe Hydrochlorid. Umkristallisation aus Methanol/Aether ergibt weisse Nadeln vom Smp. 173-174°.

Beispiel 14

Methode A: Die Herstellung von 3-Amino-1-[4-chlor-2-(2-fluorbenzoyl) phenyl] propin erfolgt auf die gleiche Weise (Methode A) wie die Herstellung von 3-Amino-1-[4-chlor-2-benzoylphenyl] propin und ergibt gelbe Prismen vom Smp. 89-91°.

Methode B: Eine Mischung aus 50 g 1-[4-Chlor-2-(2-fluorbenzoyl) phenyl]-3-phthalimidopropin, 50 ml 40 %iger Methylamin-Lösung und 150 ml Dimethylformamid wird während 25 Minuten bei Raumtemperatur gerührt. Tropfenweise gibt man 500 ml Wasser hinzu und filtriert den entstandenen Niederschlag ab. Der Niederschlag wird in Methylenchlorid gelöst, über Natriumsulfat getrocknet und im Vakuum eingedampft, wobei ein schwach gelber Festkörper anfällt. Durch Umkristallisation von 3-Amino-1-[4-chlor-2-(2-fluorbenzoyl) phenyl] propin aus Aether erhält man schwach gelbe Prismen vom Smp. 89-91°, welche in allen Belangen identisch mit authentischem Material sind.

Methode C : Eine Mischung aus 400 g (0,96 Mol) 1-[4-Chlor-2-(2-fluorbenzoyl) phenyl]-3-phthalimidopropin, 1,3 1 Aethanol und 300 ml 40 %iger Methylamin-Lösung wird bei Raumtemperatur während 2 Stunden gerührt. Tropfenweise gibt man 2,8 l Wasser hinzu und filtriert den entstandenen Niederschlag ab, wobei ein hellgelber Festkörper vom Smp. 79-80° anfällt. Durch Umkristallisation aus Aether erhält man 3-Amino-1-[4-chlor-2-(2-fluorbenzoyl) phenyl]-propin als schwach gelbe Prismen vom Smp. 89-91°, welche in allen Belangen identisch mit authentischem Material sind.

## Beispiel 15

Die Herstellung von 3-Amino-1-[4-chlor-2-(2-chlorbenzoyl) phenyl] propin erfolgt auf die gleiche Weise wie die Herstellung von 3-Amino-1-[4-chlor-2-benzoylphenyl]-propin (Methode A). Dabei erhält man schwach gelbe Prismen vom Smp. 81-82°.

## Beispiel 16

Die Herstellung von 3-Amino-1-[2-(2-chlorbenzoy)-phenyl] propin erfolgt auf die gleiche Weise wie die Herstellung von 3-Amino-1-[4-chlor-2-benzoylphenyl] propin (Methode A) und ergibt ein gelbbraunes Oel.

Das Hydrochlorid von 3-Amino-1-[2-(2-chlorbenzoyl)-phenyl] propin erhält man, indem man eine Lösung von 3-Amino-1-[2-(2-chlorbenzoyl) phenyl] propin in Methanol mit einem Ueberschuss an 6 % iger methanolischer Salzsäure versetzt und das Produkt durch Zugabe von Aether ausfällt. Durch Umkristallisation aus Methanol/Aether erhält man das reine Hydrochlorid als weisse Nadeln vom Smp. 160-162°.

## Beispiel 17

Die Herstellung von 3-Amino-1-[2-benzoylphenyl]-propin erfolgt auf die gleiche Weise wie die Herstellung von 3-Amino-1-[4-chlor-2-benzoylphenyl] propin (Methode A) und ergibt ein gelbbraunes Oel.

Durch Versetzen einer Lösung von 3-Amino-1-[2-benzoylphenyl] propin in Methanol mit einem Ueberschuss an 6 % iger methanolischer Salzsäure und anschliessendem Ausfällen mit Aether erhält man das entsprechende Hydrochlorid. Umkristallisation aus Methanol/Aether ergibt weisse Nadeln vom Smp. 157-158°.

## Beispiel 18

Eine Mischung aus 5,8 g (16 mMol) 3-Hydroxy-1-[4-chlor-2-(2-fluorbenzoyl) phenyl] propinmethansulfonat und 50 ml methanolischer Hydroxylamin-Lösung (aus 6,1 g, 88 mMol Hydroxylaminhydrochlorid) in 50 ml Tetrahydrofuran wird bei Raumtemperatur während 13 Stunden gerührt. Die Mischung wird im Vakuum eingedampft, der Rückstand wird zwischen Methylenchlorid und Wasser verteilt. Nach Trocknen der organischen Phase über Natriumsulfat und Eindampfen im Vakuum erhält man ein gelbbraunes Oel, welches 3-Hydroxyamino-1-[4-chlor-2-(2-fluorbenzoyl)-phenyl] propin enthält.

## Beispiel 19

Methode A : Eine Lösung von 14,4 g (50 mMol) 3-Amino-1-[4-chlor-2-(2-fluorbenzoyl) phenyl] propin in 50 ml Methylenchlorid wird zu einer auf 0° gekühlten Lösung von 3,0 g (10 mMol) Quecksilbersulfat in 50 ml Ameisensäure gegeben. Die Mischung wird während 3 Tagen bei 0° gerührt, auf Eis gegossen, mit Ammoniumhydroxid basisch gestellt und mit Methylenchlorid extrahiert. Die organische Phase wird über Natriumsulfat getrocknet und im Vakuum zur Trockne eingedampft. Das entstandene braune Oel löst man in 25 ml Isopropanol und gibt 4,8 g (50 mMol) Methansulfonsäure hinzu. Der entstandene Niederschlag wird abfiltriert, dabei erhält man das Methansulfonsäuresalz von 8-Chlor-1-(2-fluorphenyl)-3,4-dihydro-5H-2-benzazepin-5-on als weissliche Kristalle. Durch Umkristallisation aus Methylenchlorid/Isopropanol erhält man das Methansulfonsäuresalz als schwach gelbe Stäbe vom Smp. 176-177°.

Eine Probe des Methansulfonsäuresalzes wird zwischen Methylenchlorid und gesättigter Natriumbicarbonat-Lösung verteilt. Nach Trocknen der organischen Phase über Natriumsulfat und Eindampfen im Vakuum wird der dabei entstandene Rückstand aus Aether kristallisiert. Durch Umkristallisation aus Aether erhält man weissliche Prismen vom Smp. 109-110°.

Methode B : Eine Lösung von 200 g (0,69 Mol) 3-Amino-1-[4-chlor-2(2-fluorbenzoyl) phenyl] propin in 500 ml Methylenchlorid wird bei 5° tropfenweise zu 400 ml konzentrierter Schwefelsäure gegeben. Die Mischung wird während 3 Stunden bei 5° gerührt, auf Eis gegossen, mit Ammoniumhydroxid basisch gestellt und mit Methylenchlorid extrahiert. Die organische Phase wird über Natriumsulfat getrocknet und im Vakuum eingedampt, dabei erhält man ein braunes Oel. Das braune Oel wird in 350 ml einer 2M methanolischen Lösung von Methansulfonsäure gelöst und das entstandene Salz durch Zugabe von Aether ausgefällt. Das rohe Methansulfonsäuresalz von 8-Chlor-1-(2-fluorphenyl)-3,4-dihydro-5H-2-benzazepin-5-on wird aus Methylenchlorid/Isopropanol umkristallisiert und ergibt weissliche Stäbe vom

Smp. 176-177°, welche in allen Belangen mit authentischem Material identisch sind.

Beispiel 20

Die Herstellung von 8-Chlor-1-phenyl-3,4-dihydro-5H-2-benzazepin-5-on erfolgt auf die gleiche Weise wie die Herstellung von 8-Chlor-1-(2-fluorphenyl)-3,4-dihydro-5H-2-benzapin-5-on (Methode A) und ergibt das entsprechende Methansulfonsäuresalz als weissliche Prismen vom Smp. 187-190°.

Beispiel 21

Die Herstellung von 8-Chlor-1-(2-chlorphenyl)-3,4-dihydro-5H-2-benzazepin-5-on erfolgt auf die gleiche Weise wie die Herstellung von 8-Chlor-1-(2-fluorphenyl)-3,4-dihydro-5H-2-benzazepin-5-on (Methode A) und ergibt das entsprechende Methansulfonsäuresalz als farblose Nadeln vom Smp. 180-181°.

Beispiel 22

Die Herstellung von 1-(2-Chlorphenyl)-3,4-dihydro-5H-2-benzazepin-5-on erfolgt auf die gleiche Weise wie die Herstellung von 8-Chlor-1-(2-fluorphenyl)-3,4-dihydro-5H-2-benzazepin-5-on (Methode A) und ergibt einen schwach gelben Festkörper vom Smp. 135-137°.

Beispiel 23

Die Herstellung von 1-Phenyl-3,4-dihydro-5H-2-benzazepin-5-on erfolgt auf die gleiche Weise wie die Herstellung von 8-Chlor-1-(2-fluorphenyl)-3,4-dihydro-5H-2-benzazepin-5-on (Methode A) und ergibt das entsprechende Methansulfonsäuresalz als weissliche Prismen vom Smp. 196-198°.

Beispiel 24

Eine Lösung von 100 g (0,35 Mol) 3-Amino-1-[4-chlor-2-(2-fluorbenzoyl) phenyl] propin in 200 ml Methylenchlorid wird bei 5° tropfenweise zu 210 ml 95 %iger Schwefelsäure gegeben. Die Mischung wird während 3,5 Stunden bei 5° gerührt. Man gibt die dunkle, viskose Mischung auf 2,5 l zerstossenes Eis, stellt mit 525 ml konzentrierter Ammoniumhydroxid-Lösung basisch und extrahiert mit Methylenchlorid. Die organische Phase wird mit Wasser gewaschen, über Natriumsulfat getrocknet und im Vakkum eingedampft. Dabei erhält man ein braunes Oel, das 8-Chlor-1-(2-fluorphenyl)-3,4-dihydro-5H-2-benzazepin-5-on enthält.

Beispiel 25

Eine Lösung von 3,4 g des gemäss Beispiel 18 erhaltenen, 3-Hydroxyamino-1-[4-chlor-2-(2-fluorbenzoyl) phenyl] propin enthaltenden gelbbraunen Oels in 70 ml Methylenchlorid wird tropfenweise zu einer auf 0 °C gekühlten Mischung aus 0,7 g (2,3 mMol) Quecksilbersulfat und 17 ml Ameisensäure gegeben. Man rührt die Mischung bei Raumtemperatur über Nacht, giesst auf Eis und stellt mit Ammoniumhydroxid basisch. Die Methylenchlorid-Lösung wird mit Wasser gewaschen, über Natriumsulfat getrocknet und im Vakuum eingedampft. Reinigung des so erhaltenen gelben Oels durch Säulenchromatographie (Silicagel, 50 g ; 3 : 1 Methylenchlorid/Aether als Elutionsmittel) ergibt braune Kristalle vom Smp. 165-167°. Umkristallisation aus Aether/Methylenchlorid ergab 8-Chlor-1-(2-fluorphenyl)-3,4-dihydro-5H-2-benzazepin-5-on-2-oxid als schwach gelbe Prismen vom Smp. 167-170°, die in allen Belangen identisch mit authentischem Material sind.

Beispiel 26

Eine Mischung aus 20 g (50 mMol) 1-[4-Chlor-2-benzoylphenyl]-3-phthalimidopropin, 1,0 g (3 mMol) Quecksilbersulfat und 55 ml Ameisensäure in 50 ml Methylenchlorid wird während 30 Minuten bei Raumtemperatur gerührt. Man gibt die Mischung auf Eis und extrahiert mit Essigester. Die organische Phase wird über Natriumsulfat getrocknet und im Vakuum zur Trockne eingedampft. Der Rückstand wird aus Essigester/Aether kristallisiert und ergibt einen farblosen Festkörper. Durch Umkristallisation von 1-[4-Chlor-2-benzoylphenyl]-3-phthalimidopropan-1-on aus Aceton erhält man farblose Prismen vom Smp. 163-164°.

Eine Mischung aus 2,1 g (5 mMol) 1-[4-Chlor-2-benzoylphenyl]-3-phthalimidopropan-1-on und 10 ml 40 % iger Methylamin-Lösung in 25 ml Aethanol wird bei Raumtemperatur während 45 Minuten gerührt. Anschliessend giesst man die Mischung auf Wasser und schüttelt mit Aether aus. Die ätherische Phase wird über Natriumsulfat getrocknet und im Vakuum zur Trockne eingedampft. Der Rückstand (1,4 g) wird über eine kurze Silicagel-Kolonne filtriert, unter Nachspülen mit Methylenchlorid. Das so erhaltene Oel wird mit einer 1 M Lösung von Methansulfonsäure in Methanol behandelt und ergibt das Methansulfonsäuresalz von 8-Chlor-1-phenyl-3,4-dihydro-5H-2-benzazepin-5-on vom Smp. 187-190°. Das Produkt ist in allen Belangen identisch mit authentischem Material.

Beispiel 27

Eine Mischung aus 6,4 g (22 mMol) 8-Chlor-1-(2-fluorphenyl)-3,4-dihydro-5H-2-benzazepin-5-on und 6,4 g (34 mMol) m-Chlorperbenzoesäure in 350 ml Methylenchlorid wird bei Raumtemperatur während 2 Stunden gerührt. Die Methylenchlorid-Lösung wird mit gesättigter Natriumbicarbonat-Lösung und Wasser gewaschen, über Natriumsulfat getrocknet und im Vakuum eingedampft. Das so erhaltene gelbe Oel wird aus Aether/Petroläther umkristallisiert und ergibt weissliche Prismen vom Smp. 166-168°. Umkristallisation aus Aether/Methylenchlorid ergibt 8-Chlor-1-(2-fluorphenyl)-3,4-dihydro-5H-2-benzazepin-5-on-2-oxid als farblose Prismen vom Smp. 168-170°.

Beispiel 28

Die Herstellung von 8-Chlor-1-(2-chlorphenyl)-3,4-dihydro-5H-2-benzazepin-5-on-2-oxid erfolgt auf die gleiche Weise wie die Herstellung von 8-Chlor-1-(2-fluorphenyl)-3,4-dihydro-5H-2-benzazepin-5-on-2-oxid und ergibt gelbe Prismen vom Smp. 184-187°.

Beispiel 29

Methode A : Eine Mischung aus 7,2 g (25 mMol) 8-Chlor-1-(2-fluorphenyl)-3,4-dihydro-5H-2-benzazepin-5-on und 50 ml Dimethylformamid-dimethylacetal wird während einer Stunde zum Sieden erhitzt. Die Mischung wird im Vakuum eingedampft und ergibt braune Kristalle. Durch Umkristallisation aus Aether erhält man 8-Chlor-1-(2-fluorphenyl)-3,4-dihydro-4-[(dimethylamino)methylen]-5H-2-benzazepin-5-on als gelbe Prismen vom Smp. 228-233°.

Methode B : Eine Mischung aus 10 g (35 mMol) rohem 8-Chlor-3,4-dihydro-1-(2-fluorphenyl)-5H-2-benzazepin-5-on und 10 g (84 mMol) Dimethylformamid-dimethylacetal acetal in 10 ml Dimethylformamid wird bei Raumtemperatur während 12 Stunden gerührt. Der erhaltene Niederschlag wird abfiltriert und nacheinander mit Aethanol und Aether gewaschen. Man erhält 8-Chlor-1-(2-fluorphenyl)-3,4-dihydro-4-[(dimethylamino)methylen]-5H-2-benzazepin-5-on als braune Kristalle, welche in allen Belangen mit authentischem Material identisch sind.

Methode C : Das gemäss Beispiel 24 erhaltene Oel wird in 200 ml Dimethylformamid-dimethylacetal gelöst und während 15 Minuten auf dem Dampfbad erwärmt. Nach dem Abkühlen wird der entstandene Niederschlag abfiltriert und nacheinander mit Aethanol und Aether gewaschen. Das Produkt, 8-Chlor-1-(2-fluorphenyl)-3,4-dihydro-4-[(dimethylamino)methylen]-5H-2-benzazepin-5-on, wird an der Luft getrocknet und ergibt leicht braune Kristalle, welche in allen Belangen mit authentischem Material identisch sind.

Beispiel 30

Die Herstellung von 8-Chlor-1-phenyl-3,4-dihydro-4-[(dimethylamino)methylen]-5H-2-benzazepin-5-on erfolgt auf die gleiche Weise wie die Herstellung von 8-Chlor-1-(2-fluorphenyl)-3,4-dihydro-4-[(dimethylamino)methylen]-5H-2-benzazepin-5-on (Methode A) und ergibt gelbe Prismen vom Schmelzpunkt 180-183°.

Beispiel 31

Eine Mischung aus 18,6 g (61 mMol) 8-Chlor-1-(2-chlorphenyl)-3,4-dihydro-5H-2-benzazepin-5-on und 149 ml Dimethylformamid-dimethylacetal wird während 12 Stunden bei 50 °C gehalten. Die Mischung wird im Vakuum zur Trockne eingedampft, der Rückstand wird aus Aether/Methylenchlorid umkristallisiert und gibt 8-Chlor-1-(2-chlorphenyl)-3,4-dihydro-4-[(dimethylamino)methylen]-5H-2-benzazepin-5-on als gelben Festkörper vom Smp. 170-171°. Durch Umkristallisation aus Aether erhält man gelbe Prismen vom Smp. 170-171°.

Beispiel 32

Eine Mischung aus 3,4 g (12,5 mMol) 1-(2-Chlorphenyl)-3,4-dihydro-5H-2-benzazepin-5-on und 28 ml Dimethylformamid-dimethylacetal wird während 2 Stunden zum Sieden erhitzt. Die Mischung wird im Vakuum eingedampft, der dabei erhaltene Festkörper wird mit Aether zerrieben und gibt einen braunen Stoff vom Smp. 155-157°. Umkristallisation aus Methylenchlorid/Aether ergibt 1-(2-Chlorohenyl)-3,4-dihydro-4-[(dimethylamino)methylen]-5H-2-benzazepin-5-on als gelbe Prismen vom Smp. 158-159°.

Beispiel 33

Eine Mischung aus 5,2 g (22 mMol) 3,4-Dihydro-1-phenyl-5H-2-benzazepin-5-on und 43 ml Dimethylformamiddimethylacetal werden während 4 Stunden zum Sieden erhitzt. Die Mischung wird anschliessend im Wakuum zur Trockne eingedampft. 3,4-Dihydro-4-[(dimethylamino)methylen]-5H-2-benzazepin-5-on kristallisiert aus Aether und ergibt einen gelben Festkörper vom Smp. 131-133°. Durch Umkristallisation aus Aether erhält man gelbe Prismen vom Smp. 131-132°.

Beispiel 34

Eine Mischung aus 3,4 g (11 mMol) 8-Chlor-1-(2-fluorphenyl)-3,4-dihydro-5H-2-benzazepin-5-on-2-oxid und 26 ml Dimethylformamid-dimethylacetal wird während 12 Stunden bei Raumtemperatur gerührt. Nach Verdünnen der Mischung mit Aether und Filtrieren erhält man einen gelben Festkörper vom Smp. 175-178°. Umkristallisation aus Aether/Essigester ergibt 8-Chlor-1-(2-fluorphenyl)-3,4-dihydro-4-[(dimethylamino)methylen]-5H-2-benzazepin-5-on-2-oxid als gelbe Nadeln vom Smp. 193-194°.

Beispiel 35

Die Herstellung von 8-Chlor-1-(2-chlorphenyl)-3,4-dihydro-4-[(dimethylamino)-methylen]-5H-2-benzazepin-5-on-2-oxid erfolgt auf die gleiche Weise wie die Herstellung von 8-Chlor-1-(2-fluorphenyl)-3,4-dihydro-4-[(dimethylamino)-methylen]-5H-2-benzazepin-5-on-2-oxid und ergibt gelbe Prismen vom Smp. 196-198°.

**Ansprüche**

1. Verfahren zur Herstellung von Verbindungen der allgemeinen Formel

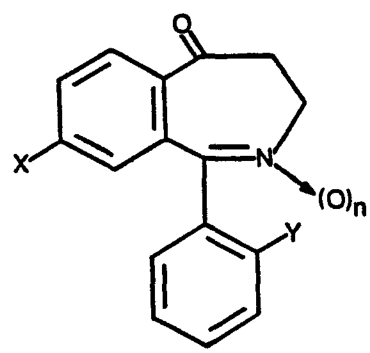

(I)

worin X und Y je Wasserstoff, Halogen mit einer Atomnummer von höchstens 35 oder Trifluormethyl und n 0 oder 1 bedeuten,
dadurch gekennzeichnet, dass man eine Verbindung der allgemeinene Formel

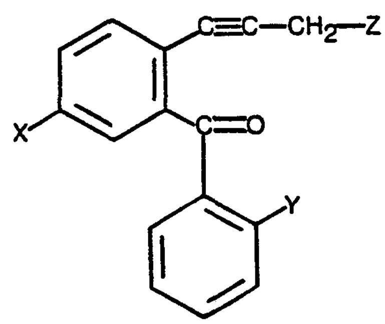

(II)

worin X und Y obige Bedeutung besitzen und Z eine Hydroxyamino-, eine Amino- oder eine geschützte Aminogruppe bedeutet,
unter sauren Bedingungen mit einem Quecksilber-(II)-Salz oder in Gegenwart von Wasser mit einer starken Säure behandelt, falls Z eine geschützte Aminogruppe ist, die Schutzgruppe abspaltet und, falls eine Verbindung der Formel I, worin n 0 ist, erhalten wird, diese Verbindung erwünschtenfalls zu einer Verbindung der Formel I, worin n I ist, oxidiert.

2. Verfahren nach Anspruch 1, dadurch gekennzeichnet, dass Z eine Hydroxyamino-, eine Amino- oder eine Phthalimidogruppe ist, dass man

a) wenn Z Hydroxyamino ist, die Verbindung der Formel II in einem inerten Lösungsmittel mit einer Mischung von Quecksilbersulfat und einer Carbonsäure ($C_1$ bis $C_5$) zur Reaktion bringt bzw.

b) wenn Z Amino ist, die Verbindung der Formel II in Gegenwart eines halogenierten Kohlenwasserstoffs als Lösungsmittel mit einer Mischung von Quecksilbersulfat und einer Carbonsäure ($C_1$ bis $C_4$) oder mit konzentrierter Schwefelsäure zur Reaktion bringt bzw.

c) wenn Z Phthalimido ist, die Verbindung der Formel II in Gegenwart eines halogenierten Kohlenwasserstoffs als Lösungsmittel mit einer Mischung von Quecksilbersulfat und einer Carbonsäure ($C_1$ bis $C_4$) zur Reaktion bringt, wonach man das so erhaltene Produkt in einem mit Wasser mischbaren Lösungsmittel mit einem primären Alkylamin oder mit Hydrazin zur Reaktion bringt
und dass man die fakultative Oxydation einer Verbindung der Formel I, worin n 0 ist, mittels einer Persäure in einem inerten organischen Lösungsmittel durchführt.

3. Verbindungen der allgemeinen Formel

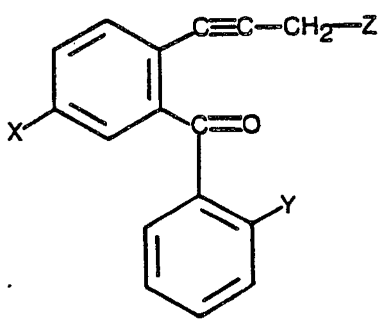

(II)

worin X, Y und Z die in Anspruch 1 angebene Bedeutung besitzen.

4. Verbindungen gemäss Anspruch 3, worin Z eine Phthalimidogruppe bedeutet.

**Claims**

1. Process for the manufacture of compounds of the general formula

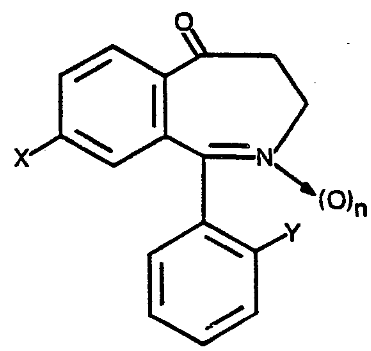

(I)

wherein X and Y each signify hydrogen, halogen with an atomic number of at most 35 or trifluoromethyl and n signifies 0 or 1,
characterized by treating a compound of the general formula

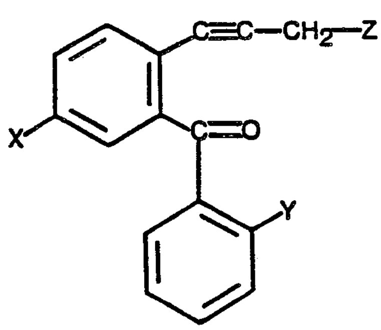

(II)

wherein X and Y have the above significance and Z signifies a hydroxyamino, an amino or a protected amino group,
under acidic conditions with a mercury (II) salt or in the presence of water with a strong acid, where Z is a protected amino group cleaving off the protecting group and, where a coumpound of formula I wherein n

is 0 is obtained, if desired oxidizing this compound to a compound of formula I wherein n is 1.

2. Process according to claim 1, characterized in that Z is a hydroxyamino, an amino or a phthalimido group, in that

a) when Z is hydroxyamino, the compound of formula II is reacted in an inert solvent with a mixture of mercuric sulphate and a carboxylic acid ($C_1$ to $C_5$) or

b) when Z is amino, the compound of formula II is reacted in the presence of a halogenated hydrocarbon as the solvent with a mixture of mercuric sulphate and a carboxylic acid ($C_1$ to $C_4$) or with concentrated sulphuric acid or

c) when Z is phthalimido, the compound of formula II is reacted in the presence of a halogenated hydrocarbon as the solvent with a mixture of mercuric sulphate and a carboxylic acid ($C_1$ to $C_4$), whereafter the thus-obtained product is reacted in a water-miscible solvent with a primary alkylamine or with hydrazine.

and in that the optional oxidation of a compound of formula I wherein n is 0 is carried out by means of a peracid in an inert organic solvent.

3. Compounds of the general formula

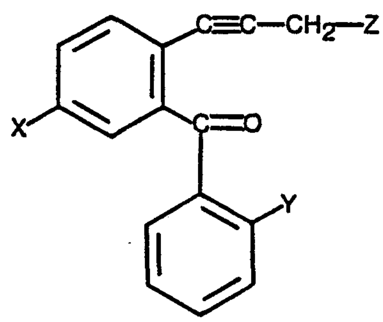

(II)

wherein X, Y and Z have the significance given in claim 1.

4. Compounds according to claim 3, wherein Z signifies a phthalimido group.

**Revendications**

1. Procédé de préparation de composés de formule générale

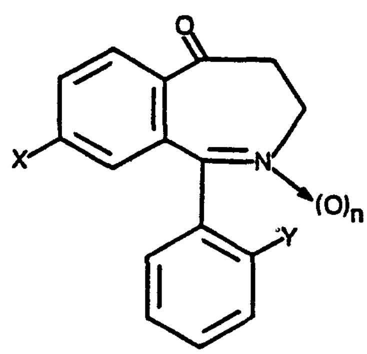

(I)

dans laquelle X et Y représentent chacun l'hydrogène, un halogène de numéro atomique 35 au maximum ou le groupe trifluorométhyle et n est égal à 0 ou 1,
caractérisé en ce que l'on traite un composé de formule générale

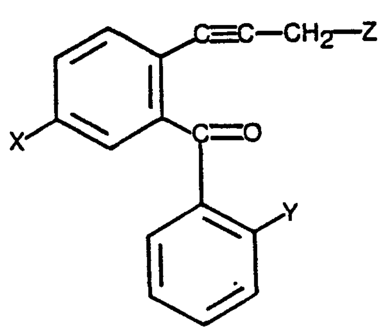

(II)

dans laquelle X et Y ont les significations indiquées ci-dessus et Z représente un groupe hydroxyamino, un groupe amino ou un groupe amino protégé,
en milieu acide par un sel de mercure -II ou en présence d'eau par un acide fort, lorsque Z est un groupe amino protégé, on élimine le groupe protecteur et lorsqu'on a obtenu un composé de formule I dans laquelle n est égal à 0, on oxyde si on le désire ce composé en un composé de formule I dans laquelle n est égal à 1.

2. Procédé selon la revendication 1, caractérisé en ce que Z est un groupe hydroxyamino un groupe amino ou un groupe phtalimido et en ce que

a) lorsque Z est un groupe hydroxyamino, on fait réagir le composé de formule II dans un solvant inerte avec un mélange de sulfate de mercure et d'un acide carboxylique (en C1-C5), ou bien

b) lorsque Z est un groupe amino, on fait réagir le composé de formule II en présence d'un hydrocarbure halogéné servant de solvant avec un mélange de sulfate de mercure et d'un acide carboxylique (en C1-C4) ou avec l'acide sulfurique concentré, ou bien

c) lorsque Z est un groupe phtalimido, on fait réagir le composé de formule II en présence d'un hydrocarbure halogéné servant de solvant avec un mélange de sulfate de mercure et d'un acide carboxylique (en C1-C4), après quoi on fait réagir le produit obtenu dans un solvant miscible à l'eau avec un alkylamine primaire ou avec l'hydrazine,
et on procède à l'oxydation facultative d'un composé de formule I dans laquelle n est égal à 0 à l'aide d'un peracide dans un solvant organique inerte.

3. Composés de formule générale

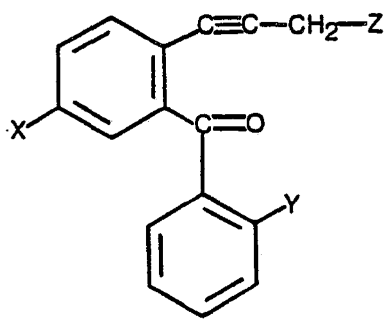

(II)

dans laquelle X, Y et Z ont les significations indiquées dans la revendication 1.

4. Composés selon la revendication 3, dans lesquels Z représente un groupe phtalimido.